# EUROPEAN PATENT APPLICATION

(11) **EP 3 118 331 A1**
(43) Date of publication of application: **18.01.2017**
(21) Application number: 16179734.5
(22) Date of filing: 15.07.2016
(51) Int. Cl.: C12Q 1/68, G01N 33/53

(54) **PARTIALLY NEUTRAL SINGLE-STRANDED OLIGONUCLEOTIDE**

(30) Priority: 15.07.2015 US 201562192987 P
(71) Applicant: OriZhan Bioscience Limited, Taipei City 110 (TW)
(72) Inventor: Wai-Hong, Chan, Hardy, Redwood City, CA California 94065 (US); Yuh-Shyong, Yang, 300 Hsinchu (TW); Wen-Yih, Chen, 320 Taoyuan City (TW)
(74) Representative: Fuchs Patentanwälte Partnerschaft mbB

(57) **Abstract**

The present invention relates to a detection unit comprising a solid surface and a partially neutral single-stranded oligonucleotide comprising a first portion. The first portion is attached to the solid surface; the length of the first portion is about 50% of the total length of the partially neutral single-stranded oligonucleotide; and the first portion comprises at least one neutral nucleotide and at least one unmodified nucleotide. The invention improves the detection sensitivity and accuracy of the detection of biomolecules.

## Description

### FIELD OF THE INVENTION

This invention relates to a molecular detection technique. More particularly, the invention relates to a partially neutral single-stranded oligonucleotide.

### BACKGROUND OF THE INVENTION

Molecular detection plays an important role in clinical diagnosis and molecular biology research. Several systems have been developed to perform molecular detection for detecting and/or identifying a target biomolecule in a sample. Among them, single-stranded oligonucleotides are used in the detection and/or identification of a target with specific nucleotide sequence based on Watson-Crick base-pairing rules.

In detail, the detection and/or identification of the target usually involves the ability to distinguish a single base variant. Commonly, in order to perform multiple detections and/or identifications in one manipulation, several single-stranded oligonucleotides are coated on one microarray substrate for hybridizing a fluorescence-labeled sample mixture. In such microarray, the operation condition such as temperature for hybridization of each single-stranded oligonucleotide in each detection and/or identification must be accommodated. Moreover, the sensitivity and specificity is critical to the microarray avoiding any false signal.

The most applicable approach to enhance sensitivity and specificity is increasing the temperature for hybridization, which is highly related to the melting temperature (*Tₘ*) of the single-stranded oligonucleotide. The melting temperature is defined as the temperature at which half of the oligonucleotide strands are in the random coil or single-stranded state. The melting temperature depends on the length of the oligonucleotide and its specific nucleotide sequence.

Several modified oligonucleotides have been developed to increase the melting temperature. For example, a peptide nucleic acid (PNA) has been disclosed (Nielsen PE, Egholm M, Berg RH, Buchardt O, 1991. "Sequence-selective recognition of DNA by strand displacement with a thymine-substituted polyamide." Science 254 (5037): 1497-500), and the backbone of PNA comprises repeating N-(2-aminoethyl)-glycine units linked by peptide bonds. The various purine and pyrimidine bases are linked to the backbone by a methylene bridge (-CH₂-) and a carbonyl group (-(C=O)-). Since the backbone of PNA contains no negatively charged phosphate groups, the binding between PNA and DNA strands is stronger than between DNA and DNA strands due to the lack of electrostatic repulsion. Unfortunately, this also causes it to be rather hydrophobic, which makes it difficult to apply in a solution state. Furthermore, in view of PNA carrying peptide bonds instead of phosphate bonds, the structural flexibility of PNA is restricted because the peptide bonds have characteristics of double bonds. It is difficult to achieve a stable conformation when binding PNA with different target molecules for various applications.

A morpholino, also known as a morpholino oligomer and as a phosphorodiamidate morpholino oligomer (PMO), has also been developed (Summerton, J; Weller D, 1997. "Morpholino Antisense Oligomers: Design, Preparation and Properties." Antisense & Nucleic Acid Drug Development 7 (3): 187-95). The structure of PMO has a backbone of methylenemorpholine rings and phosphorodiamidate linkages. Because of completely unnatural backbones, PMO cannot not be recognized by cellular proteins such as enzymes, and it restricts the applications involved in protein binding as well as enzyme recognition.

A locked nucleic acid (LNA) is provided as a modified RNA nucleotide (Satoshi Obika; Daishu Nanbu; Yoshiyiki Hari; Ken-ichiro Morio; Yasuko In; Toshimasa Ishida; Takeshi Imanishi, 1997. "Synthesis of 2'-O,4'-C-methyleneuridine and -cytidine. Novel bicyclic nucleosides having a fixed C3'-endo sugar puckering." Tetrahedron Lett. 38 (50): 8735-8). The ribose moiety of an LNA nucleotide is modified with an extra bridge connecting the 2' oxygen and 4' carbon. The bridge "locks" the ribose in the 3'-endo (North) conformation, which is often found in the A-form duplexes. The locked ribose conformation enhances base stacking and backbone pre-organization. This significantly increases the melting temperature of oligonucleotides. However, the popularity of LNA is limited by its chemistry of synthesis and the price of having LNA designed and synthesis.

US 2014/0235465 A1 discloses a neutralized DNA (nDNA). All of the charged oxygen ions (O⁻) on the phosphate backbone have been alkylated, so that the backbone is totally electrically neutral. This modification increases the hybridization efficiency between complementary single stranded DNAs and minimizes the salt required for hybridization, which results in higher sensitivity of the detection. However, nDNA applied to hybridization does not produce satisfactory specificity, and nonspecific hybridization occurs in salt concentrations for regular DNA hybridization conditions.

A field-effect transistor (FET) has been employed to detect and/or identify a target oligonucleotide as well as an antibody-antigen binding, both of which benefit from the absence of labeling requirements for reagents and the ready availability of commercial manufacturing sources for FET sensors. Sensitivity of the FET sensor is highly dependent on detection distance (debye length) between the transistor surface and the actual detected molecules. Most current types of FET are less than satisfactory as gene detection devices in terms of sensitivity. This is mainly due to the requirement of relatively high salt concentrations for DNA/DNA or DNA/RNA hybridizations. Hybridization of highly charged biomolecules requires an appropriate ionic strength to suppress the charge repulsive forces. Unfortunately, ions in the hybridization buffer also reduce the FET debye length and hence diminish detection sensitivity. In the case of antibody-antigen binding detection, the large size of the antibody as compared to other biomolecules also reduces the detection sensitivity of the FET. The surface charges distribution of antibody and the binding orientation of the bound antibody make FET detection difficulty to be resolved and be quantitatively analyzed. In addition, medium concentrations of salt in the binding buffer also reduce the debye length and, in turn, lower the detection sensitivity as well.

### SUMMARY OF THE INVENTION

In order to improve detection sensitivity and specificity, a labeling-free detection method is provided.

The invention is to provide a partially neutral single-stranded oligonucleotide comprising at least one electrically neutral nucleotide and at least one negatively charged nucleotide.

The present invention is also to provide a detection unit comprising the partially neutral single-stranded oligonucleotide as mentioned above.

The present invention is also to provide a detection system comprising a plurality of the detection units as mentioned above.

The present invention is also to provide a method of detection comprising performing the detection with the detection unit as mentioned above.

The present invention is described in detail in the following sections. Other characteristics, purposes and advantages of the present invention can be found in the detailed description and claims.

### BRIEF DESCRIPTION OF THE DRAWINGS

FIG. 1 shows one preferred embodiment of the electrically neutral nucleotide according to the invention.
FIG. 2 shows a schematic drawing of using the partially neutral single-stranded oligonucleotide in FET for hybridization detection.
FIG. 3 shows a schematic drawing of using the partially neutral single-stranded oligonucleotide as a recovery probe in FET for hybridization detection.
FIG. 4 shows a schematic drawing of using the partially neutral single-stranded oligonucleotide as a recovery probe and nanogold particles as a signal amplifier in FET for hybridization detection.
FIG. 5 shows the result of the ionic strength effects of the partially neutral single-stranded oligonucleotide according to the invention in FET.
FIG. 6 shows the result of the partially neutral single-stranded oligonucleotide according to the invention in FET in detecting different target concentrations.
FIG. 7 shows the result of the partially neutral single-stranded oligonucleotide according to the invention in FET in detecting ultralow target concentrations.
FIGs. 8A and 8B show the results of the polymerase chain reaction using DNA (8 A) and partially neutral single-stranded oligonucleotide according to the invention (8 B).
FIG. 9 shows the results of the quantitative polymerase chain reaction using DNA and partially neutral single-stranded oligonucleotide according to the invention.
FIGs. 10A to 10C show the results of using DNA (miR-524-5p DNA probe) to detect miRNA by *in situ* hybridization. FIG. 10A shows cells expressing negative miRNAs and miR-524-5p DNA probe with 1.5 ng/µl concentration. FIG. 10B and FIG. 10C show cell expressing mimic miR-524-5p and miR-524-5p probe with the concentration of 0.3 ng/µl (FIG. 10 B) and 1.5 ng/µl (FIG. 10 C).
FIGs. 11A to 11C show the results of using nDNA (miR-524-5p All nDNA probe) to detect miRNA by *in situ* hybridization. FIG. 11A shows cells expressing negative miRNAs and miR-524-5p nDNA probe with 1.5 ng/µl concentration. FIG. 11B and FIG. 11C show cells expressing mimic miR-524-5p and miR-524-5p probe with the concentration of 0.3 ng/µl (FIG. 11 B) and 1.5 ng/µl (FIG. 11 C).
FIGs. 12A to 12C show the results of using partially neutral single-stranded oligonucleotide (miR-524-5p 7nDNA probe) to detect miRNA by *in situ* hybridization. FIG. 12A shows cells expressing negative miRNAs and miR-524-5p 7nDNA probe with 1.5 ng/µl concentration. FIG. 12B and FIG. 12C show cells expressing mimic miR-524-5p and probe with concentration of 0.3 ng/µl (FIG. 12 B) and 1.5 ng/µl (FIG. 12 C).
FIGs. 13A to 13C show the results of using LNA (miR-524-5p LNA probe) to detect miRNA by *in situ* hybridization. FIG. 13A shows Cells expressing negative miRNAs and miR-524-5p LNA probe with 1.5 ng/µl concentration. FIG. 13 B and FIG. 13C show cells expressing mimic miR-524-5p and probe with concentration of 0.3 ng/µl (FIG. 13 B) and 1.5 ng/µl (FIG. 13 C).
FIG. 14 shows the result of the partially neutral single-stranded oligonucleotide according to the invention in miRNA detection by FET.

### DETAILED DESCRIPTION OF THE INVENTION

The invention is to provide a partially neutral single-stranded oligonucleotide comprising at least one electrically neutral nucleotide and at least one negatively charged nucleotide.

As used herein, the term "oligonucleotide" refers to a nucleotide oligomer. The term "nucleotide" refers to an organic molecule composed of a nitrogenous base, a sugar, and one or more phosphate groups; preferably one phosphate group. The nitrogenous base includes a derivative of purine or pyrimidine. The purine includes substituted or unsubstituted adenine and substituted or unsubstituted guanine; the pyrimidine includes substituted or unsubstituted thymine, substituted or unsubstituted cytosine and substituted or unsubstituted uracil. The sugar is preferably a five-carbon sugar, more preferably substituted or unsubstituted ribose or substituted or unsubstituted deoxyribose. The phosphate groups form bonds with the 2, 3, or 5-carbon of the sugar; preferably, with the 5-carbon site. For forming the oligonucleotide, the sugar of one nucleotide is joined to the adjacent sugar by a phosphodiester bridge. A proper form of the partially neutral single-stranded oligonucleotide can be chosen as needed based on the disclosure of the invention. Preferably, the partially neutral single-stranded oligonucleotide is DNA or RNA; more preferably DNA.

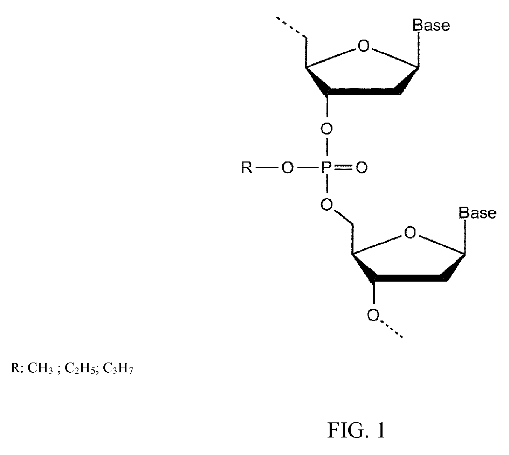

The partially neutral single-stranded oligonucleotide according to the invention comprises at least one electrically neutral nucleotide and at least one negatively charged nucleotide. The manner of rendering a nucleotide electrically neutral is not limited. In one embodiment of the invention, the electrically neutral nucleotide comprises a phosphate group substituted by an alkyl group. Preferably, the alkyl group is a C₁-C₆ alkyl group; more preferably, the alkyl group is a C₁-C₃ alkyl group. Examples of the C₁-C₃ alkyl group include but are not limited to methyl, ethyl and propyl. FIG. 1 shows one preferred embodiment of the electrically neutral nucleotide according to the invention. The negatively-charged oxygen atom in the phosphate group is changed to a neutral atom without charge. The way to substitute the phosphate group with the alkyl group can be applied according to common chemical reactions.

The negatively charged nucleotide according to the invention comprises a phosphate group with at least one negative charge. The unmodified nucleotide is preferably a naturally occurring nucleotide without modification or substitution. In one preferred embodiment of the invention, the negatively charged nucleotide comprises an unsubstituted phosphate group.

The partially neutral single-stranded oligonucleotide according to the invention is partially rendered electrically neutral. The sequence or length is not limited, and the sequence or length of the partially neutral single-stranded oligonucleotide can be designed according to a target molecule based on the disclosure of the invention.

The number of the electrically neutral nucleotides and negatively charged nucleotides depend on the sequence of the partially neutral single-stranded oligonucleotide and the condition under which a reaction is to be detected. The positions of the electrically neutral nucleotide and negatively charged nucleotide also depend on the sequence of the partially neutral single-stranded oligonucleotide and the condition under which a reaction is to be detected. The number and position of the electrically neutral nucleotides and negatively charged nucleotides can be designed according to the available information based on the disclosure of the invention. For example, the number and position of the electrically neutral nucleotides in any probes can be designed by molecular modeling calculation based on double stranded (ds) structural energy, and the melting temperature (Tm) of dsDNA/DNA or dsDNA/RNA can then be determined by reference to the structural energy.

In one preferred embodiment of the invention, the partially neutral single-stranded oligonucleotide comprises a plurality of the electrically neutral nucleotides, and at least one negatively charged nucleotide is positioned between two of the electrically neutral nucleotides; more preferably, at least two negatively charged nucleotides are positioned between two of the electrically neutral nucleotides.

In one preferred embodiment of the invention, when applying the partially neutral single-stranded oligonucleotide according to the invention in the detection and/or identification of a single base variant, at least one electrically neutral nucleotide is positioned near the single base variant; more preferably, 2, 3, 4, or 5 electrically neutral nucleotides are positioned near the single base variant. In another aspect, at least one electrically neutral nucleotide is positioned at the downstream or upstream site of the single base variant; preferably, at least one electrically neutral nucleotide is positioned at the downstream and upstream sites of the single base variant.

By introducing the electrically neutral nucleotide, the melting temperature difference between perfect match double-stranded oligonucleotides and mismatched double-stranded oligonucleotides of the partially neutral single-stranded oligonucleotide according to the invention is higher compared with that of a conventional DNA probe. Without being restricted by theory, it is surmised that the electrostatic repulsion force between two strands is lowered by introducing the neutral oligonucleotide, and the melting temperature is raised thereby. By controlling the number and position of electrically neutral nucleotides, the melting temperature difference is adjusted to a desired point, providing a better working temperature or temperature range to differentiate the perfect and mismatched oligonucleotides, thereby improving the detection specificity. Such design benefits the consistency of the melting temperature of different partially neutral single-stranded oligonucleotides integrated in one chip or array. The number of reactions to be detected can be raised dramatically with high specificity and more detection units can be incorporated into a single detection system. The design provides better microarray operation conditions.

The present invention is also to provide a detection unit comprising the partially neutral single-stranded oligonucleotide as mentioned above.

As used herein, the term "detection" refers to a process of discovering or determining the existence or presence of a target molecule, and preferably, a process of identifying the target molecule. In one preferred embodiment of the invention, the detection comprises quantifying the target molecule in a sample. A reaction applied in the detection includes but is not limited to hybridization between oligonucleotide molecules, protein-protein interaction, receptor-ligand binding, oligonucleotide-protein interaction, polysaccharide-protein interaction, or small molecule-protein interaction.

The present invention is also to provide a detection system comprising a plurality of the detection units as mentioned above.

As used herein, the term "unit" refers to a component for carrying out the reaction applied in the detection. Preferably, the unit is for carrying out a single reaction. In a preferred embodiment of the invention, several units are contained in one system to carry out several detections in one manipulation. For example, a plurality of detection units may be incorporated in a detection system. Preferably, the detection system is a microarray or a chip.

As used herein, the term "molecule" refers to a small molecule or a macromolecule. Preferably, the molecule is a macromolecule such as a protein, peptide, nucleotide, oligonucleotide or polynucleotide. The molecule is naturally occurring or artificial. In another aspect, the molecule is purified or mixed with other contents. In one preferred embodiment of the invention, the expression pattern of the molecule is different in a normal condition and in an abnormal condition, such as a disease. In another preferred embodiment of the invention, the expression pattern of the molecule is different in different cell types. In yet another preferred embodiment of the invention, the molecules are DNA molecules, RNA molecules, antibodies, antigens, enzymes, substrates, ligands, receptors, cell membrane-associated proteins or cell surface markers. The DNA molecules are preferably a gene or an untranscripted region. The RNA molecules are preferably mRNA, micro RNA, long untranslated RNA, rRNA, tRNA, or siRNA.

The term "target molecule" used herein refers to a specified molecule to be detected or identified from a pool of molecules.

The sample according to the invention is derived from a naturally occurring origin or derived from an artificial manipulation. Preferably, the sample is derived from a naturally occurring origin such as an extract, body fluid, tissue biopsy, liquid biopsy, cell culture. In another aspect, the sample is processed according to the reaction required for detection. For example, the pH value or ion strength of the sample may be adjusted.

The partially neutral single-stranded oligonucleotide according to the invention in the detection unit may be presented in a solution or linked to a support. In one preferred embodiment of the invention, the detection unit further comprises a solid surface; and the partially neutral single-stranded oligonucleotide is attached on or located near the solid surface.

As used herein, the term "solid surface" refers to a solid support including but not limited to a polymer, paper, fabric, or glass. The solid surface to be employed varies depending on the reaction signal to be detected. For example, when the detection adopts a field-effect transistor to monitor the signal, the solid surface is a transistor surface of the field-effect transistor; when the detection adopts a surface plasmon resonance, the solid surface is a metal surface of a surface plasmon resonance; when the detection adopts a microarray detection system, the solid surface is a substrate surface of the microarray.

In a preferred embodiment of the invention, the material of the solid surface is silicon; preferably polycrystalline silicon or single crystalline silicon; more preferably polycrystalline silicon. Polycrystalline silicon is cheaper than single crystalline silicon, but because the polycrystalline has more grain boundary, a defect usually occurs in the grain boundary that hinders electron transduction. Such phenomenon makes the solid surface uneven and quantification difficult. Furthermore, ions may penetrate into the grain boundary of the polycrystalline and cause detection failure in solution. In addition, polycrystalline silicon is not stable in air. The abovementioned drawbacks, however, would not interfere with the function of the detection unit according to the invention.

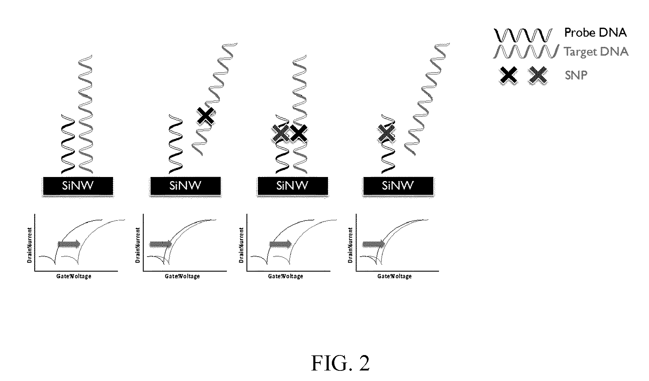

In one embodiment of the invention, the detection unit further comprises a signal detection component. The signal detection component is applied for detecting whether hybridization and/or binding occurs between the partially neutral single-stranded oligonucleotide and the target molecule. Preferably, the detection component is a field-effect transistor, a surface plasmon resonance, a microscope, a spectrometer, an electrophoresis device, or an electrochemical sensor. In one embodiment of the invention, referring to FIG. 2, when the target molecule of DNA or RNA hybridizes with the partially neutral single-stranded oligonucleotide to form a complex, the charge changes compared to that of the partially neutral single-stranded oligonucleotide because the target molecule of DNA or RNA carries abundant negative charges. If a mismatch occurs between the partially neutral single-stranded oligonucleotide and the target molecule such as single nucleotide polymorphism detection, no complex forms. The signal detection component is used for detecting the charge change and monitoring if hybridization occurs. The signal detection component is usually integrated with the solid surface for detecting the voltage of the solid surface. Artisans skilled in this field are able to design the detection component according to the invention.

In one preferred embodiment of the invention, the partially neutral single-stranded oligonucleotide comprises a first portion attached to the solid surface; the length of the first portion is about 50% of the total length of the partially neutral single-stranded oligonucleotide; and the first portion comprises at least one electrically neutral nucleotide and at least one negatively charged nucleotide; more preferably, the length of the first portion is about 40% of the total length of the partially neutral single-stranded oligonucleotide; still more preferably, the length of the first portion is about 30% of the total length of the partially neutral single-stranded oligonucleotide.

In one preferred embodiment of the invention, the partially single-stranded nucleotide further comprises a second portion adjacent to the first portion. The second portion is located in the distal end relative to the solid surface. The second portion comprises at least one electrically neutral nucleotide and at least one negatively charged nucleotide. The description of the electrically neutral nucleotide and the negatively charged nucleotide is the same as that of the first portion and is not repeated herein.

The manner of attaching the partially neutral single-stranded oligonucleotide and the solid surface depends on the material of the solid surface and the type of the partially neutral single-stranded oligonucleotide. In one embodiment of the invention, the partially neutral single-stranded oligonucleotide links to the solid surface through a covalent bond. Examples of the covalent bond include but are not limited to the following methods, depending on the solid surface chemistry and the modification of the oligonucleotide. In one embodiment of the invention, when using silicon oxide as the solid surface, the solid surface is modified by using (3-Aminopropyl)triethoxysilane (APTES). The silicon atom in the molecule of APTES performs a covalent bond with the oxygen atom of the hydroxyl group and it converts the surface's silanol groups (SiOH) to amines; then the 5'-amino group of partially neutral single-stranded oligonucleotide is covalently bonded with the solid surface amines group by glutaraldehyde (Roey Elnathan, Moria Kwiat, Alexander Pevzner, Yoni Engel, Larisa Burstein Artium Khatchtourints, Amir Lichtenstein, Raisa Kantaev, and Fernando Patolsky, Biorecognition Layer Engineering: Overcoming Screening Limitations of Nanowire-Based FET Devices, Nano letters, 2012, 12, 5245-5254). In one another embodiment of the invention, the solid surface is modified into self-assembly monolayer molecules with different functional groups for covalently linking to different functional groups of the partially neutral single-stranded oligonucleotide by various chemical reactions (Srivatsa Venkatasubbarao, Microarrays - status and prospects, TRENDS in Biotechnology Vol.22 No. 12 December 2004; Ki Su Kim, Hyun-Seung Lee, Jeong-A Yang, Moon-Ho Jo and Sei Kwang Hahn, The fabrication, characterization and application of aptamer-functionalized Si-nanowire FET biosensors, Nanotechnology 20 (2009)).

In one another preferred embodiment of the invention, the partially neutral single-stranded oligonucleotide is located near the solid surface. In view that the detection component is applied for monitoring the voltage change of the solid surface, the partially neutral single-stranded oligonucleotide is not necessary to directly bind to the solid surface, provided that the distance between the partially neutral single-stranded oligonucleotide and the solid surface is short enough allowing the detection component to detect the voltage change. Preferably, the distance between the solid surface and the partially neutral single-stranded oligonucleotide is about 0 to about 10 nm; more preferably about 0 to about 5 nm.

In one preferred embodiment of the invention, the detection unit according to the invention further comprises a signal amplifier. The signal amplifier according to the invention preferably refers to a component that enhances the detection of the voltage change of the solid surface. For example, the signal amplifier is nanogold particles attached to one end of the partially neutral single-stranded oligonucleotide.

In one preferred embodiment of the invention, the detection unit further comprises a buffer with an ionic strength lower than about 50 mM; more preferably lower than about 40 mM, 30 mM, 20 mM or 10 mM. Without being restricted by theory, it is surmised that by applying the partially neutral single-stranded oligonucleotide, the hybridization between the partially neutral single-stranded oligonucleotide with the target DNA or RNA molecule can happen without the need to suppress the electrostatic repulsive forces between the partially charged semi-neutral single-stranded oligonucleotide and its target. The hybridization is then driven by the base pairing and the stacking force of each strand. Consequently, hybridization can be performed at a lower salt condition. With FET, the lower ion strength increases the detection length (the debye length) and, in turn, enhances the detection sensitivity.

In one preferred embodiment of the invention, the detection unit further comprises a biomolecule for expanding the application of the detection unit. Examples of the biomolecule include but are not limited to a single-stranded DNA molecule, a single-stranded RNA molecule, a polypeptide or a protein.

In one more preferred embodiment of the invention, the detection unit further comprises a protein linked to the partially neutral single-stranded oligonucleotide. Artisans skilled in this field can complete the linkage between the protein and the partially neutral single-stranded oligonucleotide, for example, by specific affinity binding. Through linking to various proteins, the detection system serves as a protein chip with improved hybridization specificity for detecting a target interaction with the linked protein.

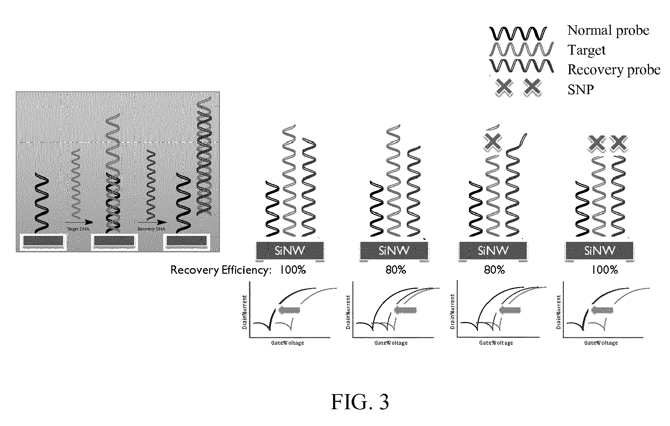

In another embodiment of the invention, referring to FIG. 3, the detection unit further comprises a single-stranded DNA as a normal probe, and the partially neutral single-stranded oligonucleotide serves as a recovery probe. The single-stranded DNA as the normal probe may be a modified signal-stranded DNA or an unmodified single-stranded DNA; preferably, a partially neutral single-stranded DNA. The recovery probe and the normal probe both have binding affinity to a target; more preferably, the recovery probe has higher affinity to the target than the normal probe. The recovery probe competes with the normal probe in a complex formed with the normal probe and the target. Such a displacement can be readily observed, for example, with the field-effect transistor. In one embodiment of the invention, the normal probe is designed to detect the presence of the target, and the recovery probe is design to detect the presence of single nucleotide polymorphism with a mismatched nucleotide. The normal probe is first to form a complex with the target, and the recovery probe is then bound to the target in the complex. The signal is monitored to detect if a mismatch is present between the recovery probe and the target.

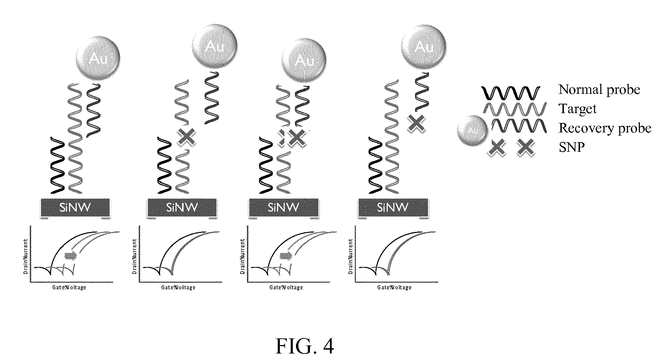

In one more preferred embodiment of the invention, referring to FIG. 4, the recovery probe links to nanogold particles for amplifying the signal.

The present invention is also to provide a method of detection comprising performing the detection with the detection unit as mentioned above.

Various applications of the method according to the invention are provided. The detection includes but is not limited to non-labeling gene expression, polymerase chain reaction, in situ hybridization, single nucleotide polymorphism (SNP) detection, RNA detection, DNA conjugated protein and protein detection.

Particularly, by applying the detection unit according to the invention, a very small amount of the target molecule can be detected in the sample without amplifying the target molecule in a polymerase chain reaction. The detection preferably comprises detecting a target of less than 10⁻⁹ Molar in the sample, and the method is free of a polymerase chain reaction.

In one embodiment of the invention, the improved hybridization specificity in gene detection can be seen mainly in two aspects of FET detection compared to a conventional detection. First, the melting temperature difference between perfect match and mismatch is higher. Second, the buffer has a lower salt condition, and the FET detection length (the debye length) is increased. Both of these differences result in improvement of detection sensitivity.

In still another embodiment of the present invention, the partially neutral single-stranded oligonucleotide designed on the field effect transistor microarray or surface plasmon resonance microarray provides quantitative gene expression, protein and SNP detection in a non-labelled and a high throughput fashion.

The following examples are provided to aid those skilled in the art in practicing the present invention.

### EXAMPLES

### Example 1: Synthesis of partially neutral single-stranded oligonucleotide

Deoxy cytidine (n-ac) p-methoxy phosphoramidite, thymidine p-methoxy phosphoramidite, deoxy guanosine (n-ibu) p-methoxy phosphoramidite, and deoxy adenosine (n-bz) p-methoxy phosphoramidite (all purchased from ChemGenes Corporation, USA)were used to synthesize an oligonucleotide according to a given sequence based on solid-phase phosphotriester synthesis or by Applied Biosystems 3900 High Throughput DNA Synthesizer (provided by Genomics® Biosci & Tech or Mission Biotech).

The synthesized oligonucleotide was reacted with weak alkaline in toluene at room temperature for 24 hours, and the sample was subjected to ion-exchange chromatography to adjust the pH value to 7. After the sample was concentrated and dried, the partially neutral single-stranded oligonucleotide was obtained.

### Example 2: Partially neutral single-stranded oligonucleotide applied in FET detection Complementary H1:

5'-CCATTGTGACTGTCCTCAAGTAGGTTGACAGAGTGTG-3' (SEQ ID No. 1)
Noncomplementary H5: 5'-TGATAACCAATGCAGATTTG-3' (SEQ ID No. 2)
DNA probe: 5'-NH₂-C6-CACACTCTGTCAACCTAC-3' (SEQ ID No. 3)
nDNA probe: 5'-NH₂-C6-CACACTCTGTCAACCTAC-3'(All neutral) (SEQ ID No. 4)
Partially neutral single-stranded probe 1 (hereafter referred to as "modified 1"): 5'-NH₂-C6-CⁿACⁿACⁿTCⁿTGⁿTCAACCTAC-3' (SEQ ID No. 5)
Partially neutral single-stranded probe 2 (hereafter referred to as "modified 2"): 5'-NH₂-C6-CⁿACAⁿCTCⁿTGTⁿCAACCTAC-3' (SEQ ID No. 6)
Superscript n represents electrically neutral nucleotide.

### Surface modification for probe immobilization

Probe immobilization was performed by functionalization of the SiNW surface layer (SiO₂). First, (3-Aminopropyl)triethoxysilane (APTES) was used to modify the surface. The silicon atom in the molecule of APTES performed a covalent bond with the oxygen of the hydroxyl group and converted the surface's silanol groups (SiOH) to amines. Samples were immersed in 2% APTES (99% EtOH) for 30 minutes and then heated to 120°C for 10 min. After this step, amino groups (NH₂) were the terminal units from the surface.

Next, glutaraldehyde was used as a grafting agent for DNA immobilization. Glutaraldehyde binding was achieved through its aldehyde group (COH) to ensure a covalent bond with the amino group of APTES. For this step, samples were immersed in 12.5% glutaraldehyde (10mM sodium phosphate buffer) in liquid for 1 hour at room temperature. For probe immobilization, 5'-amino group of DNA strands were linked to the aldehyde groups of the linker. A 500 µL drop solution of 1 µmol DNA probes was deposited onto the NWs for 18 hours.

### Target DNA hybridization and sensing

After probe immobilization, PDMS (polydimethylsiloxane) fluidic system was developed for pumping DNA targets to the nanowire surface to hybridize to the DNA probes. Complementary and non-complementary targets were used, with various concentrations with dilution with bis-tris propane [1,3-bis(tris(hydroxymethyl)methylamino)propane] solution. After 30 min for hybridization, samples were washed with bis-tris buffer for 10 min to remove excess targets. Finally, Keithley 2400 was used to detect the NWFET electrical characteristics (Id vs. Vg curves).

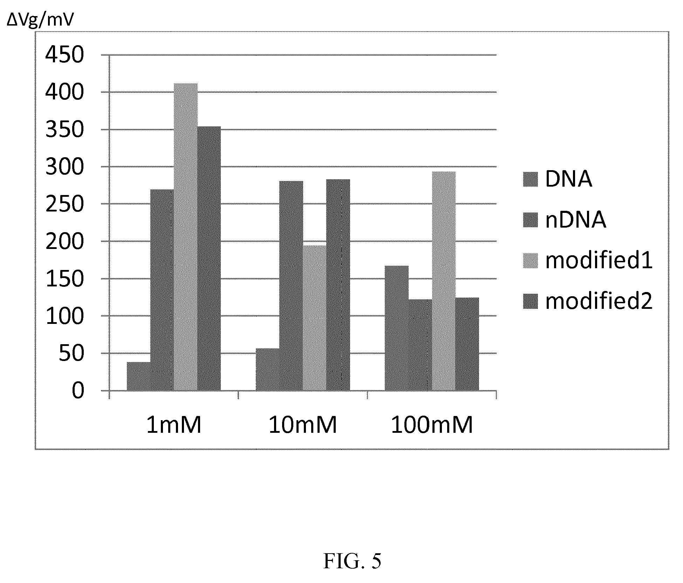

The results of ionic strength effects are shown in FIG 5. nDNA and partially neutral single-stranded DNA in low salt concentrations have higher FET sensitivity in ΔV of DNA in high salt concentration. Partially neutral single-stranded DNA with 3' end modifications has the highest FET sensitivity.

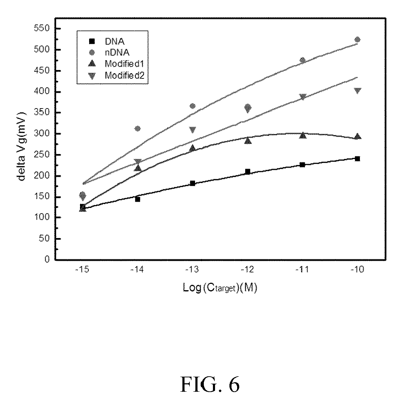

The results of detecting different target concentrations at 1 mM bis-tris are shown in FIG 6. nDNA and partially neutral single-stranded DNA probes have higher concentration sensitivity (slope of V. vs. Conc.), especially in low target concentration ranges.

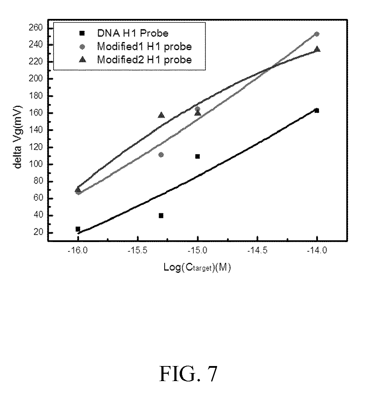

The results of detecting ultralow target concentrations at 1 mM bis-tris are shown in FIG 7. The target concentration can be detected as low as 0.1 fM with partially neutral single-stranded DNA probe on FET.

The specificity was assayed if the non-specific hybridization with H5T occurred. The results are shown in Table 1.

**Table 1:**

| | **H5T ΔV/mV** | **H1T ΔV/mV** |
|---|---|---|
| DNA (in 100 mM) | 73.64mV | |
| nDNA (in 1 mM) | 218.1mV | 269.3mV |
| modified1 (n-X-n) (in 1 mM) | 39.11mV | 173.34mV |
| modified2 (n-X-X-n) (in 1 mM) | 25.8mV | 205.11mV |

As indicated in Table 1, the Δ V/mV of H5T (indicating nonspecific hybridization) is higher when using nDNA as probe. The nonspecific hybridization is reduced when using the partially neutral DNA probes in low salt concentration. Example 3: Partially neutral single-stranded oligonucleotide applied in polymerase chain reaction

The partially neutral single-stranded oligonucleotides are applied in polymerase chain reaction (PCR) and quantitative polymerase chain reaction (qPCR) of SYBR™ Green system.
Template: pUC19
Product length: 202bp

**Table 2: Primer sequence**

| Name | Sequences (5' to 3') | SEQ ID No. |
|---|---|---|
| FP_R | TTAGCTCACTCATTAGGCA C | 7 |
| RP_R | GGGCCTCTTCGCTATTACG C | 8 |
| FP_R_m12 | CTCACTCCTTAGGCAC | 9 |
| FP_n10,14 | CTCACTⁿCATTⁿAGGCAC | 10 |
| FP_m12_n10,14 | CTCACTⁿCCTTⁿAGGCAC | 11 |
| FP_n11,13 | CTCACTCⁿATⁿTAGGCAC | 12 |
| FP_m12_n11,13 | TTAGCTCACTC^nCT^nTAGGC AC | 13 |

Superscript n represents electrically neutral nucleotide; "_" represents mismatched nucleotide

**Table 3: PCR program:**

| Step | Temperature (°C) | Duration | Cycle number |
|---|---|---|---|
| Initial denaturation | 94 | 2 min | 1 |
| Denaturation | 94 | 30 sec | 30 |
| Annealing | gradient | 15 sec | |
| Extension | 72 | 30 sec | |
| Final extension | 72 | 5 min | 1 |

**Table 4: qPCR Program:**

| Step | Temperature (°C) | Duration | Cycle number |
|---|---|---|---|
| Initial denaturation | 95 | 7 min | 1 |
| denaturation | 95 | 10 sec | 40 |
| Annealing | gradient | 15 sec | |
| Extension | 60 | 15 sec | |

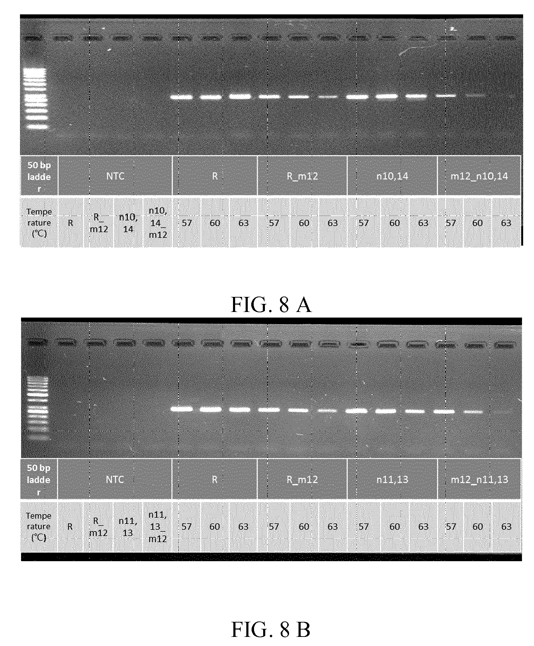

The results of PCR are shown in FIGs. 8A and 8B. Comparing the results of using DNA and those of using the partially neutral single-stranded oligonucleotides (FIG. 8 A: Regular DNA primer (FP-R and RP-R of Table 2) vs. neutralized DNA modifications primer (FP-n 10, 14 of Table 2) ;FIG. 8 B: Regular DNA primer(FP-R and RP-R of Table 2) vs. neutralized DNA modifications primer (FP-n 11, 13 of Table 2)), the primer specificity of the partially neutral single-stranded oligonucleotides is significantly improved.

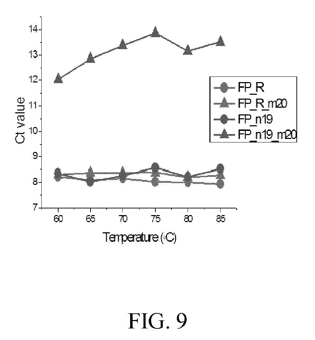

The results of qPCR are shown in FIG. 9 and Table 5.

**Table 5:**

| Annealing temperature | 60°C | ΔCt | 65°C | ΔCt | 70°C | ΔCt | 75°C | ΔCt | 80°C | ΔCt | 85 C | ΔCt |
|---|---|---|---|---|---|---|---|---|---|---|---|---|
| FP_R | 8.22 | 0.291 | 8.079 | 0.291 | 8.151 | 0.214 | 8.013 | 0.375 | 8.009 | 0.197 | 7.944 | 0.34 |
| FP_R_m20 | 8.297 | | 8.371 | | 8.365 | | 8.388 | | 8.205 | | 8.289 | |
| FP_n19 | 8.348 | 3.681 | 8.024 | 1.828 | 8.256 | 5.114 | 8.569 | 5.295 | 8.223 | 1.936 | 8.521 | 1.994 |
| FP_n19_m20 | 12.029 | | 12.852 | | 13.371 | | 13.864 | | 13.159 | | 13.515 | |
| Fold (nDNA/regular) | | 47.19 | | 16.566 | | 23.853 | | 14.127 | | 25.115 | | 14.467 |

Comparing the results of using DNA and those of using the partially neutral single-stranded oligonucleotides, the primer specificity of the partially neutral single-stranded oligonucleotides is significantly improved.

### Example 4: Partially neutral single-stranded oligonucleotide applied in detecting miRNA by in situ hybridization

Test cells: SK MEL19 cells transfected with mimic miR-524-5p and SK MEL19 cells transfected with miR-67 (negative control)
Probes:
   Negative control miRNAs (Cel-miR-67): TCACAACCTCCTAGAAAGAGTAGA (SEQ ID No. 14)
   Mimic miR-524-5p: CUACAAAGGGAAGCACUUUCUC (SEQ ID No. 15)
   MiR-524-5p LNA probe: EX-38630-05- LNA Detection probe, hsa-miR-524-5p, 250 pmol 3'-DIG labeled MiR-524-5p 7N-DNA probe: 5'-GAⁿGAAⁿAGTⁿGCTⁿTCCⁿCTTⁿTGTⁿAG-3'Dig (SEQ ID No. 16) Superscript n represents electrically neutral nucleotide.
   MiR-524-5p All nDNA probe: 5' GAGAAAGTGCTTCCCTTTGTAG/3'Dig/ (SEQ ID No. 17)
   MiR-524-5p DNA probe: 5'-GAGAAAGTGCTTCCCTTTGTAG-3'Dig (SEQ ID No. 18)

The *in situ* hybridization were performed the manufacture's instructions of ISHyb *in situ* hybridization kit (BioChain, Newark, CA, USA).The test cells were immobilized with 4% paraformaldehyde (DEPC-PBS) for 20 minutes and then washed with DEPC-PBS three times (5 minutes each time). The test cells were treated with proteinase (10 µg/ml) for 8 minutes and washed with DEPC-PBS for 5 minutes. The cells were further immobilized with 4% PFA (DEPC-PBS) for 15 minutes then washed with DEPC-PBS three times (5 minutes each time). The washed test cells were treated with prehybridization solution (BioChain, Newark, CA, USA) at 65°C for 4 hours. Different concentrations of probes were then added for reacting at 55°C for 12 to 16 hours. The samples were washed with 2×SSC at 45 °C for 10 minutes, 1.5 ×SSC at 45 °C for 10 minutes, and then 0.2×SSC at 37°C for 20 minutes. The hybridization was blocked with 1×blocking solution (BioChain, Newark, CA, USA) for 1 hour. An antibody solution (1:200) (BioChain, Newark, CA, USA)_was added for reacting for 4 hours, and the test cells were washed with DEPC-PBS three times (10 minutes each time) and then with alkaline phosphatase buffer (BioChain, Newark, CA, USA) twice (5 minutes each). NBT/BCIP (nitro blue tetrazolium/5-bromo-4-chloro-3indolyl phosphate) 6.6µl NBT + 3.3µl BCIP) solution was then added for 2 to 20 hours. The samples were washed with water and observed under the microscope.

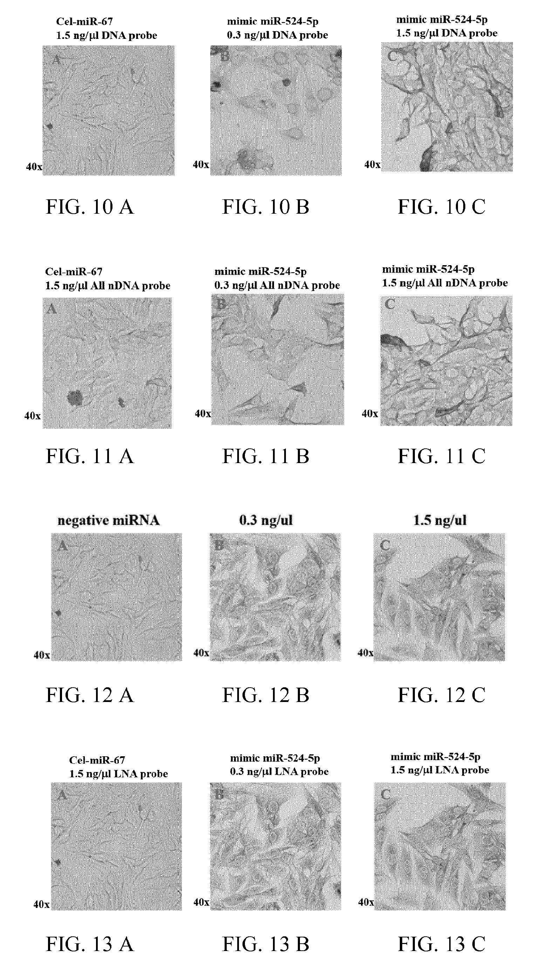

The results are shown in FIGs. 10A to 13C. The higher concentration of probes can produce better staining image than the lower concentration of probes in these four different probes (DNA, all nDNA, 7nDNA, LNA). These results indicate that all four probes can detect specifically miR-524-5p expression in cells. It is noted that the 7nDNA probe produces the most sensitivity to miR-524-5p expression than LNA, all nDNA and DNA probes, because the stronger intensity image is observed when cells are applied in 7nDNA probes compared with the same concentration of LNA or nDNA probes.

### Example 5: Partially neutral single-stranded oligonucleotide applied in detecting miRNA

Target miRNA:
miR107 (1pM): AGCAGCAUUGUACAGGGCUAUCA (SEQ ID No. 19)
miR579-3p (1pM): UUCAUUUGGUAUAAACCGCGAUU (SEQ ID No. 20)
miR885-5p (1pM): UCCAUUACACUACCCUGCCUCU (SEQ ID No. 21) Probes are shown in Table 6.

**Table 6:**

| **Name** | **Sequences (5' to 3')** | **SEQ ID No.** |
|---|---|---|
| probe 107 (DNA) | NH₂-C6-TGATAGCCCTGTACAATGCTGCT | 22 |
| probe 107 (modified2) | NH₂-C6-TⁿGATⁿAGCⁿCCTⁿGTACAATGCTGCT | 23 |
| probe 579-3p (DNA) | NH₂-C6-AATCGCGGTTTATACCAAATGAA | 24 |
| probe 579-3p (modified2) | NH₂-C6-AⁿATCⁿGCGⁿGTTⁿTATACCAAATGAA | 25 |
| probe 885-5p (DNA) | NH₂-C6-AGAGGCAGGGTAGTGTAATGGA | 26 |
| probe 885-5p (modified2) | NH₂-C6-AⁿGAGⁿGCAⁿGGGⁿTAGTGTAATGGA | 27 |

The surface modification for probe immobilization was as described in Example 2. One 1pM miRNA (complementary to the probe) in bis-tris propane buffer was added to the probe-immobilized chip for reacting for 30 minutes. The chip was washed with bis-tris propane buffer for 10 minutes. The signal was monitored as described in Example 2.

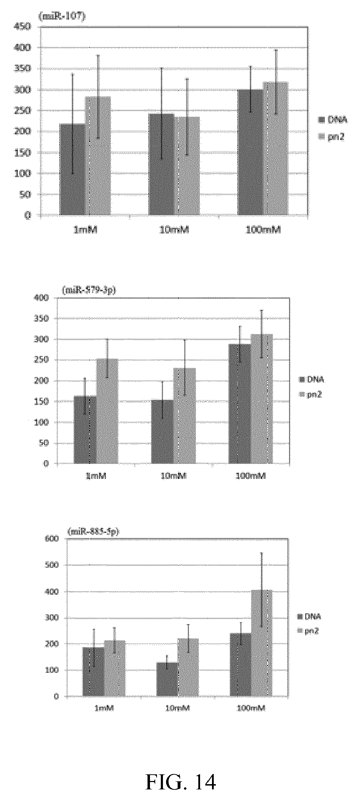

The results are shown in FIG. 14. The Δ V/mV of partially single-stranded oligonucleotide probes is higher than that of DNA probe. The partially neutral single-stranded probe has higher sensitivity.

While the present invention has been described in conjunction with the specific embodiments set forth above, many alternatives thereto and modifications and variations thereof will be apparent to those of ordinary skill in the art. All such alternatives, modifications and variations are regarded as falling within the scope of the present invention.

## Claims

1. A partially neutral single-stranded oligonucleotide comprising at least one electrically neutral nucleotide and at least one negatively charged nucleotide.

2. The partially neutral single-stranded oligonucleotide according to claim 1, wherein the electrically neutral nucleotide comprises a phosphate group substituted by a C₁-C₆ alkyl group.

3. The partially neutral single-stranded oligonucleotide according to claim 1, wherein the negatively charged nucleotide comprises an unsubstituted phosphate group.

4. The partially neutral single-stranded oligonucleotide according to claim 1, which comprises a plurality of the electrically neutral nucleotides, and at least one negatively charged nucleotide is positioned between two of the electrically neutral nucleotides.

5. A detection unit comprising the partially neutral single-stranded oligonucleotide according to claim 1.

6. The detection unit according to claim 5, which further comprises a solid surface; and the partially neutral single-stranded oligonucleotide is attached on or located near the solid surface.

7. The detection unit according to claim 6, wherein the partially neutral single-stranded oligonucleotide comprises a first portion attached to the solid surface; the length of the first portion is about 50% of the total length of the partially neutral single-stranded oligonucleotide; and the first portion comprises at least one electrically neutral nucleotide and at least one negatively charged nucleotide.

8. The detection unit according to claim 6, wherein the solid surface is a transistor surface of a field-effect transistor (FET); a metal surface of a surface plasmon resonance (SPR) or a substrate surface of a microarray.

9. The detection unit according to claim 6, wherein the material of the solid surface is polycrystalline silicon or single crystalline silicon.

10. The detection unit according to claim 5 further comprising a signal detection component.

11. The detection unit according to claim 10, wherein the detection component is a field-effect transistor, a surface plasmon resonance, a microscope, a spectrometer, an electrophoresis device, or an electrochemical sensor.

12. The detection unit according to claim 5 further comprising a signal amplifier.

13. The detection unit according to claim 12, wherein the signal amplifier is nanogold particles attached to one end of the partially neutral single-stranded oligonucleotide.

14. The detection unit according to claim 5 further comprising a buffer with the ionic strength lower than about 50 mM.

15. The detection unit according to claim 5 further comprising a biomolecule.

16. The detection unit according to claim 15, wherein the biomolecule is selected from the group consisting of a single-stranded DNA molecule, a single-stranded RNA molecule, a polypeptide and a protein.

17. A detection system comprising a plurality of the detection units according to claim 5.

18. The detection system according to claim 17, which is a microarray or a chip.

19. A method of detection comprising performing a detection with the detection unit according to claim 5.

20. The method according to claim 19, wherein the detection is selected from the group consisting of non-labeling gene expression, polymerase chain reaction, *in situ* hybridization, single nucleotide polymorphism (SNP) detection, RNA detection, DNA conjugated protein and protein detection.


SEQUENCE LISTING

<110>  OriZhan Bioscience Limited

<120>  PARTIALLY NEUTRAL SINGLE-STRANDED OLIGONUCLEOTIDE

<130>  none

<160>  27

<170>  PatentIn version 3.5

<210>  1
<211>  37
<212>  DNA
<213>  Artificial Sequence

<220>
<223>  Complementary H1

<400>  1
ccattgtgac tgtcctcaag taggttgaca gagtgtg                                37


<210>  2
<211>  20
<212>  DNA
<213>  Artificial Sequence

<220>
<223>  Noncomplementary H5

<400>  2
tgataaccaa tgcagatttg                                                   20


<210>  3
<211>  24
<212>  DNA
<213>  Artificial Sequence

<220>
<223>  DNA probe

<400>  3
cccccccaca ctctgtcaac ctac                                              24


<210>  4
<211>  24
<212>  DNA
<213>  Artificial Sequence

<220>
<223>  nDNA probe

<400>  4
cccccccaca ctctgtcaac ctac                                              24


<210>  5
<211>  24
<212>  DNA

<213>  Artificial Sequence

<220>
<223>  Partially neutral single-stranded probe 1


<220>
<221>  misc_difference
<222>  (7)..(7)
<223>  n represents electrically neutral c


<220>
<221>  misc_difference
<222>  (9)..(9)
<223>  n represents electrically neutral c


<220>
<221>  misc_difference
<222>  (11)..(11)
<223>  n represents electrically neutral c


<220>
<221>  misc_difference
<222>  (13)..(13)
<223>  n represents electrically neutral c


<220>
<221>  misc_difference
<222>  (15)..(15)
<223>  n represents electrically neutral g


<400>  5
ccccccnana ntntntcaac ctac                                              24


<210>  6
<211>  24
<212>  DNA
<213>  Artificial Sequence

<220>
<223>  Partially neutral single-stranded probe 2


<220>
<221>  misc_difference
<222>  (7)..(7)
<223>  n represents electrically neutral c


<220>
<221>  misc_difference
<222>  (10)..(10)
<223>  n represents electrically neutral a


<220>
<221>  misc_difference

<222>  (13)..(13)
<223>  n represents electrically neutral c


<220>
<221>  misc_difference
<222>  (16)..(16)
<223>  n represents electrically neutral t


<400>  6
ccccccnacn ctntgncaac ctac                                              24


<210>  7
<211>  20
<212>  DNA
<213>  Artificial Sequence

<220>
<223>  FP_R

<400>  7
ttagctcact cattaggcac                                                   20


<210>  8
<211>  20
<212>  DNA
<213>  Artificial Sequence

<220>
<223>  RP_R

<400>  8
gggcctcttc gctattacgc                                                   20


<210>  9
<211>  20
<212>  DNA
<213>  Artificial Sequence

<220>
<223>  FP_R_m12

<400>  9
ttagctcact ccttaggcac                                                   20


<210>  10
<211>  20
<212>  DNA
<213>  Artificial Sequence

<220>
<223>  FP_n10,14


<220>
<221>  misc_difference
<222>  (10)..(10)
<223>  n represents electrically neutral t

<220>
<221>  misc_difference
<222>  (14)..(14)
<223>  n represents electrically neutral t


<400>  10
ttagctcacn catnaggcac                                                    20


<210>  11
<211>  20
<212>  DNA
<213>  Artificial Sequence

<220>
<223>  FP_m12_n10,14


<220>
<221>  misc_difference
<222>  (10)..(10)
<223>  n represents electrically neutral t


<220>
<221>  misc_difference
<222>  (14)..(14)
<223>  n represents electrically neutral t


<400>  11
ttagctcacn cctnaggcac                                                    20


<210>  12
<211>  20
<212>  DNA
<213>  Artificial Sequence

<220>
<223>  FP_n11,13


<220>
<221>  misc_difference
<222>  (11)..(11)
<223>  n represents electrically neutral c


<220>
<221>  misc_difference
<222>  (13)..(13)
<223>  n represents electrically neutral t


<400>  12
ttagctcact nantaggcac                                                    20


<210>  13

<211>  20
<212>  DNA
<213>  Artificial Sequence

<220>
<223>  FP_m12_n11,13


<220>
<221>  misc_difference
<222>  (11)..(11)
<223>  n represents electrically neutral c


<220>
<221>  misc_difference
<222>  (13)..(13)
<223>  n represents electrically neutral t


<400>  13
ttagctcact ncntaggcac                                                    20


<210>  14
<211>  24
<212>  DNA
<213>  Artificial Sequence

<220>
<223>  Negative control miRNAs (Cel-miR-67)

<400>  14
tcacaacctc ctagaaagag taga                                               24


<210>  15
<211>  22
<212>  RNA
<213>  Artificial Sequence

<220>
<223>  Mimic miR-524-5p

<400>  15
cuacaaaggg aagcacuuuc uc                                                 22


<210>  16
<211>  22
<212>  DNA
<213>  Artificial Sequence

<220>
<223>  MiR-524-5p 7N-DNA probe


<220>
<221>  misc_difference
<222>  (2)..(2)
<223>  n represents electrically neutral a

<220>
<221>  misc_difference
<222>  (5)..(5)
<223>  n represents electrically neutral a


<220>
<221>  misc_difference
<222>  (8)..(8)
<223>  n represents electrically neutral t


<221>  misc_difference
<222>  (11)..(11)
<223>  n represents electrically neutral t


<220>
<221>  misc_difference
<222>  (14)..(14)
<223>  n represents electrically neutral c


<220>
<221>  misc_difference
<222>  (17)..(17)
<223>  n represents electrically neutral t


<220>
<221>  misc_difference
<222>  (20)..(20)
<223>  n represents electrically neutral t


<400>  16
gnganagngc ntcnctntgn ag                                                22


<210>  17
<211>  22
<212>  DNA
<213>  Artificial Sequence

<220>
<223>  MiR-524-5p All nDNA probe

<400>  17
gagaaagtgc ttccctttgt ag                                                22


<210>  18
<211>  22
<212>  DNA
<213>  Artificial Sequence

<220>
<223>  MiR-524-5p DNA probe

<400>  18
gagaaagtgc ttccctttgt ag                                                22

<210>  19
<211>  23
<212>  RNA
<213>  Artificial Sequence

<220>
<223>  miR107

<400>  19
agcagcauug uacagggcua uca                                              23


<210>  20
<211>  23
<212>  RNA
<213>  Artificial Sequence

<220>
<223>  miR579-3p

<400>  20
uucauuuggu auaaaccgcg auu                                              23


<210>  21
<211>  22
<212>  RNA
<213>  Artificial Sequence

<220>
<223>  miR885-5p

<400>  21
uccauuacac uacccugccu cu                                               22


<210>  22
<211>  29
<212>  DNA
<213>  Artificial Sequence

<220>
<223>  probe 107 (DNA)

<400>  22
   cccccctgat agccctgtac aatgctgct                                       29


<210>  23
<211>  29
<212>  DNA
<213>  Artificial Sequence

<220>
<223>  probe 107 (modified2)


<220>
<221>  misc_difference
<222>  (7)..(7)
<223>  n represents electrically neutral t

<220>
<221>  misc_difference
<222>  (10)..(10)
<223>  n represents electrically neutral t


<220>
<221>  misc_difference
<222>  (13)..(13)
<223>  n represents electrically neutral c


<220>
<221>  misc_difference
<222>  (16)..(16)
<223>  n represents electrically neutral t


<400>  23
ccccccngan agnccngtac aatgctgct                                         29


<210>  24
<211>  29
<212>  DNA
<213>  Artificial Sequence

<220>
<223>  probe 579-3p (DNA)

<400>  24
ccccccaatc gcggtttata ccaaatgaa                                         29


<210>  25
<211>  29
<212>  DNA
<213>  Artificial Sequence

<220>
<223>  probe 579-3p (modified2)


<220>
<221>  misc_difference
<222>  (7)..(7)
<223>  n represents electrically neutral a


<220>
<221>  misc_difference
<222>  (10)..(10)
<223>  n represents electrically neutral c


<220>
<221>  misc_difference
<222>  (13)..(13)
<223>  n represents electrically neutral g


<220>
<221>  misc_difference

<222>  (16)..(16)
<223>  n represents electrically neutral t

<400>  25
ccccccnatn gcngtntata ccaaatgaa                                         29

<210>  26
<211>  28
<212>  DNA
<213>  Artificial Sequence

<220>
<223>  probe 885-5p (DNA)

<400>  26
ccccccagag gcagggtagt gtaatgga                                          28

<210>  27
<211>  28
<212>  DNA
<213>  Artificial Sequence

<220>
<223>  probe 885-5p (modified2)

<220>
<221>  misc_difference
<222>  (7)..(7)
<223>  n represents electrically neutral a

<220>
<221>  misc_difference
<222>  (10)..(10)
<223>  n represents electrically neutral g

<220>
<221>  misc_difference
<222>  (13)..(13)
<223>  n represents electrically neutral a

<220>
<221>  misc_difference
<222>  (16)..(16)
<223>  n represents electrically neutral g

<400>  27
ccccccngan gcnggntagt gtaatgga                                          28